# EUROPEAN PATENT APPLICATION

(11) **EP 3 095 449 A1**
(43) Date of publication of application: **23.11.2016**
(21) Application number: 16160083.8
(22) Date of filing: 03.08.2009
(51) Int. Cl.: A61K 35/28, A61P 29/00

(54) **USES OF MESENCHYMAL STEM CELLS**

(30) Priority: 04.08.2008 GB 0814249
(62) Divisional of application: 09786159.5
(71) Applicant: TiGenix, S.A.U., 28760 Tres Cantos - Madrid (ES); Consejo Superior De Investigaciones Científicas, 28006 Madrid (ES); Universidad de Sevilla, 41013 Sevilla (ES)
(72) Inventor: DELGADO, Mario, 18100 Armilla (Granada) (ES); GONZALEZ-REY, Elena, Seville (ES); BÜSCHER, Dirk, 28760 Tres Cantos Madrid (ES)
(74) Representative: Carpmaels & Ransford LLP

(57) **Abstract**

The invention relates to the use of mesenchymal stem cells (MSCs) for treating systemic inflammatory response syndrome (SIRS) in a subject. The invention provides compositions, uses and methods for the treatment of SIRS.

SEQUENCE LISTING

<110>   TIGENIX, S.A.U.
        CONSEJO SUPERIOR DE INVESTIGACIONES CIENTIFICAS
        UNIVERSIDAD DE SEVILLA

<120>   USES OF MESENCHYMAL STEM CELLS

<130>   P067729EP

<140>   EP16160083.8
<141>   2009-08-03

<150>   GB0814249.9
<151>   2008-08-04

<150>   PCT/IB2009/006597
<151>   2009-08-03

<150>   EP09786159.5
<151>   2009-08-03

<160>   2

<170>   SeqWin2010, version 1.0

<210>   1
<211>   25
<212>   DNA
<213>   Artificial Sequence

<220>
<223>   IDO forward primer

<400>   1
ggattcttcc tggtctctct attgg                                          25

<210>   2
<211>   25
<212>   DNA
<213>   Artificial Sequence

<220>
<223>   IDO reverse primer

<400>   2
cggactgagg gatttgactc taatg                                          25

## Description

### BACKGROUND TO THE INVENTION

Systemic inflammatory response syndrome (SIRS) is an inflammatory state of the whole body without a specific source of infection. It can be caused by many factors, including but not limited to trauma, surgery, adrenal insufficiency, pulmonary embolism, myocardial infarction, hemorrhage, anaphylaxis, drug overdose, immunodeficiency and bums. There are four major diagnostic symptoms of SIRS, as listed below, but the presence of any two of these is sufficient for a diagnosis (see e.g. Nystrom (1998) Journal of Antimicrobial Chemotherapy, 41, Suppl. A, 1-7).
i) a heart rate in excess of 90 beats per minute;
ii) a body temperature below 36°C or above 38°C;
iii) a respiratory rate in excess of 20 breaths per minutes (tachypnea); and
vi) a white blood cell count below 4000 cells/mm³ or above 12000 cells/mm³, or the presence of more than 10% immature neutrophils.

SIRS causes widespread activation of acute phase immunogenic proteins, affecting the complement system and the coagulation pathways, which in turn cause damage to the vasculature as well as the internal organs. Various neuroendocrine counter-regulatory systems are subsequently activated, which often compound the problem.

Sepsis is a specific form of SIRS, and the most common cause of death in intensive care units. It is caused by a suspected or detected infection. It is characterized by a hyperactive and out-of-balance network of endogenous pro-inflammatory cytokines, and often leads to widespread inflammation and blood clotting, which may result in redness, heat, swelling, pain, organ dysfunction or organ failure. Blood clotting during sepsis may also cause reduced blood flow to the limbs and vital organs, and can lead to organ failure or the onset of gangrene.

Like SIRS, sepsis often results in an acute inflammation present throughout the body, and is therefore frequently associated with fever and leukocytosis (elevated white blood cell count). The modem theory behind sepsis is that the host's immune response to the infection triggers SIRS, which in turn presents the symptoms described above. Following infection and sepsis, tissue perfusion and oxygen delivery may be reduced leading to septic shock. In order to be diagnosed with septic shock, there must be evidence of infection and refractory hypotension in the patient.

SIRS, sepsis and septic shock are severe medical conditions. Even with immediate and aggressive treatment, these diseases are likely to progress to multiple organ dysfunction syndrome and may even result in death.

Most therapeutic strategies to date have targeted pro-inflammatory mediators, but they have not been found to improve survival of patients when studied in large multi-center clinical trials. Therapies designed to block one single cytokine, such as TNFα and IL-1β, have shown limited efficacy probably due to the early and transient kinetic of these inflammatory cytokines. Recently, international critical care and infectious disease experts have developed management guidelines to improve the treatment given to patients suffering from SIRS, sepsis or septic shock (Dellinger, 2004). These guidelines aim to transform complex diagnostic and therapeutic decisions into simple "mission critical" tasks and, among other treatments, suggest the administration of broad-spectrum antibiotics, steroids and Drotrecogin Alfa (Activated).

However, mortality associated with sepsis remains at 30% to 50%, whilst the mortality rate for septic shock is reported to be even higher, at 50% to 60%. There are reported to be approximately 750,000 new sepsis cases each year, with at least 210,000 of these resulting in a fatality. As medical treatments become more aggressive, the incidences of SIRS, sepsis and septic shock are likely to increase, and consequently a new reliable treatment for these conditions is required.

### SUMMARY OF THE INVENTION

It has been found that administration of mesenchymal stem cells (MSCs), in particular human adipose tissue derived stromal stem cells (hASCs), protects against mortality in two *in vivo* models of severe endotoxemia and sepsis, providing evidence that MSCs may be useful in the treatment of SIRS, sepsis and septic shock. It has been found that MSCs function at several levels to regulate crucial aspects of SIRS, including by reduction of systemic levels of various inflammatory cytokines and chemokines, and by inhibition of leukocyte infiltration into various target organs.

The invention therefore provides a composition comprising mesenchymal stem cells (MSCs), for use in treating systemic inflammatory response syndrome (SIRS) in a subject.

The invention also provides the use of mesenchymal stem cells (MSCs) in the manufacture of a medicament for treating systemic inflammatory response syndrome (SIRS) in a subject.

The invention also provides a method of treating systemic inflammatory response syndrome (SIRS) in a subject, comprising administering mesenchymal stem cells (MSCs) to the subject.

These and other aspects of the invention are described in more detail below.

### BRIEF DESCRIPTION OF THE FIGURES

Figure 1 shows cell characterization as defined by the markers expressed by the cell, and measured by immunofluorescence staining. The frequency of immunopositive cells is indicated as follows: -, less than 5%; +/-, 6-15%; +, 16-50%; ++, 51-85%; and +++, 86-100%. P, Passage number.
Figure 2 shows indirect immunofluorescence characterization of adipose tissue-derived stromal stem cells. Blue color indicates DAPI-stained nuclei. (A) CD90; (B) c-Kit; and (C) vimentin.
Figure 3 shows fluorescence immunocytometry analysis of the profile of surface markers (CD3, CD9, CD10, CD11b, CD13, CD14, CD15, CD16, CD18, CD19, CD28, CD29, CD31, CD34, CD36, CD38, CD44, CD45, CD49a, CD49b, CD49c, CD49d, CD49e, CD49f, CD50, CD51, CD54, CD55, CD56, CD58, CD59, CD61, CD62E, CD62L, CD62P, CD90, CD95, CD102, CD104, CD105, CD106, CD133, CD166, glycoforina, β2 microglobulin, HLA I, HLA II and NGFR) obtained from cells isolated from liposuction samples.
Figure 4 shows the effect of incubation with pro-inflammatory reagents on the expression of IDO in mesenchymal stem cells isolated from human adipose tissue. A) detection by RT-PCR. B) detection by western blot. IL-1, interleukin 1; TNF-α, tumour necrosis factor-alpha; LPS, lipopolysaccharide; IFN-γ, interferon-gamma; C-, negative control; C+, positive control; n.i., cells not induced with IFN-γ. GAPDH (glyceraldehyde-3-phosphate dehydrogenase) is used as loading control of the RT-PCR.
Figure 5 shows fluorescence immunocytometry analysis of the profile of surface markers expressed by ASCs. Isotype controls (negative controls) are shown shaded in grey.
Figure 6 shows the effect of stimulation of hASCs on the secretion of IFNγ and the proliferation of PBMCs.
Figure 7 shows the response of hASCs to LPS or CLP: (A) Cell survival was monitored every 12 h; (B) Cytokine and chemokine levels in liver, intestine and lung; (C) Concentration of inflammatory cells infiltrating the peritoneal cavity; (D) Neutrophil infiltration in target organs, determined by measuring MPO activity in protein extracts. n=10 mice/group. *p<0.001 vs controls with LPS alone or CLP alone.

### DETAILED DESCRIPTION OF THE INVENTION

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

The term "about" when used in relation to a value relates to the value ±10%.

By "adipose tissue" is meant any fat tissue. The adipose tissue may be brown or white adipose tissue, derived from, for example, subcutaneous, omental/visceral, mammary, gonadal, or other adipose tissue site. Preferably, the adipose tissue is subcutaneous white adipose tissue. The adipose tissue may comprise a primary cell culture or an immortalized cell line. The adipose tissue may be from any organism having fat tissue. In some embodiments, the adipose tissue is mammalian, and in further embodiments the adipose tissue is human. A convenient source of adipose tissue is liposuction surgery. However, it will be understood that neither the source of adipose tissue nor the method of isolation of adipose tissue is critical to the invention. If cells as described herein are desired for autologous transplantation into a subject, the adipose tissue will be isolated from that subject.

"Adipose tissue-derived stromal stem cells (ASCs)" refers to MSCs that originate from adipose tissue, generally from human adipose tissue (hASCs).

The term "constitutively" is understood to mean the expression of a gene without any specific induction.

The term "culture" refers to the growth of cells, organisms, multicellular entities, or tissue in a medium. The term "culturing" refers to any method of achieving such growth, and may comprise multiple steps. The term "further culturing" refers to culturing a cell, organism, multicellular entity, or tissue to a certain stage of growth, then using another culturing method to bring said cell, organism, multicellular entity, or tissue to another stage of growth. A "cell culture" refers to a growth of cells in vitro. In such a culture, the cells proliferate, but they do not organize into tissue per se. A "tissue culture" refers to the maintenance or growth of tissue, e.g., explants of organ primordial or of an adult organ in vitro so as to preserve its architecture and function. A "monolayer culture" refers to a culture in which cells multiply in a suitable medium while mainly attached to each other and to a substrate. Furthermore, a "suspension culture" refers to a culture in which cells multiply while suspended in a suitable medium. Likewise, a "continuous flow culture" refers to the cultivation of cells or explants in a continuous flow of fresh medium to maintain cell growth, e.g. viability. The term "conditioned media" refers to the supernatant, e.g. free of the cultured cells/tissue, resulting after a period of time in contact with the cultured cells such that the media has been altered to include certain paracrine and/or autocrine factors produced by the cells and secreted into the culture. A "confluent culture" is a cell culture in which all the cells are in contact and thus the entire surface of the culture vessel is covered, and implies that the cells have also reached their maximum density, though confluence does not necessarily mean that division will cease or that the population will not increase in size.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

The terms "comprise" and "comprising" are used in the inclusive, open sense, meaning that additional elements may be included.

The term "including" is used herein to mean "including but not limited to". "Including" and "including but not limited to" are used interchangeably.

"Marker" refers to a biological molecule whose presence, concentration, activity, or phosphorylation state may be detected and used to identify the phenotype of a cell.

"Mesenchymal Stem Cells (MSCs)" are multipotent stem cells, *i.e.* they are cells which are capable of giving rise to multiple different types of cells. The term refers to cells which are capable of differentiating into at least one of an osteoblast, a chondrocyte, an adipocyte, or a myocyte. MSCs may be isolated from any type of tissue. Generally MSCs will be isolated from bone marrow, adipose tissue, umbilical cord, or peripheral blood. The MSCs used in the invention may in some embodiments be isolated from bone marrow (BM-MSCs) or adipose tissue (ASCs). In a preferred aspect of the invention, MSCs are obtained from lipoaspirates, themselves obtained from adipose tissue.

A "patient", "subject" or "host" to be treated by the subject method may mean either a human or non-human animal.

The term "pharmaceutical composition" refers to a composition intended for use in therapy. The compositions of the invention are pharmaceutical compositions, intended for use in treating SIRS, sepsis, severe sepsis, septic shock and sepsis-like conditions. The compositions of the invention may include, in addition to MSCs, non-cellular components. Examples of such non-cellular components include but are not limited to cell culture media, which may comprise one or more of proteins, amino acids, nucleic acids, nucleotides, co-enzyme, anti-oxidants and metals.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

The term "phenotype" refers to the observable characteristics of a cell, such as size, morphology, protein expression, etc.

The term "progenitor cell" refers to a cell that has the capacity to create progeny that are more differentiated than itself. For example, the term may refer to an undifferentiated cell or cell differentiated to an extent short of final differentiation which is capable of proliferation and giving rise to more progenitor cells having the ability to generate a large number of mother cells that can in turn give rise to differentiated or differentiable daughter cells. In one embodiment, the term progenitor cell refers to a generalized mother cell whose descendants (progeny) specialize, often in different directions, by differentiation, e.g., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity may be natural or may be induced artificially upon treatment with various factors. By this definition, stem cells may also be progenitor cells, as well as the more immediate precursors to terminally differentiated cells.

"Proliferation" refers to an increase in cell number. "Proliferating" and "proliferation" refer to cells undergoing mitosis.

The term "systemic inflammatory response syndrome" (or "SIRS") is used herein in accordance with its normal meaning, to refer to an inflammatory state of the whole body without a source of infection. There are four major diagnostic symptoms of SIRS, although any two of these are enough for a diagnosis (see *e.g.* Nystrom (1998) Journal of Antimicrobial Chemotherapy, 41, Suppl A, 1-7).

The term "sepsis" refers to a form of SIRS which is caused by a suspected or proven infection (see *e.g.* Nystrom (1998) Journal of Antimicrobial Chemotherapy, 41, Suppl. A, 1-7). An infection that leads to sepsis may be caused by *e.g.* a virus, a fungus, a protozoan or a bacterium.

The term "severe sepsis" refers to sepsis associated with organ dysfunction, hypoperfusion or hypotension (see *e.g.* Nystrom (1998) Journal of Antimicrobial Chemotherapy, 41, Suppl. A, 1-7).

The term "septic shock" refers to sepsis with hypotension despite adequate resuscitation with fluids (refractory hypotension), along with the presence of perfusion abnormalities (see *e.g.* Nystrom (1998) Journal of Antimicrobial Chemotherapy, 41, Suppl. A, 1-7).

The term "sepsis-like condition" refers to a state in which a patient presents with symptoms similar to sepsis or septic shock but where the cascade of inflammatory mediators and/or the change in haemodynamic parameters are not primarily or initially caused by an infectious agent. For example, sepsis-like conditions may be seen in a patient with acute or chronic liver failure (see Wasmuth HE, et al. J Hepatol. 2005 Feb;42(2): 195-201), patients suffering from post-resuscitation disease after cardiac arrest (see Adrie C et al. Curr Opin Crit Care. 2004 Jun;10(3):208-12), patients suffering from sepsis-like symptoms after cancer chemotherapy (see Tsuji E et al. lnt J Cancer. 2003 Nov 1;107(2):303-8) patients undergoing hyperthermic isolated limb perfusion with recombinant TNF-alpha or similar treatments (see Zwaveling JH et al. Crit Care Med. 1996 May;24(5):765-70) or sepsis-like illness in neonates (see Griffin MP et al. Pediatr Res. 2003 Jun;53(6):920-6.

As used herein, the term "solution" includes a pharmaceutically acceptable carrier or diluent in which the MSCs used in the invention remain viable.

The term "substantially pure", with respect to MSC populations, refers to a population of cells in which at least about 75%, at least about 85%, at least about 90%, or at least about 95%, by number of the cells are MSCs. In other words, the term "substantially pure", with respect to MSC populations, refers to a population of cells that contains less than about 20%, less than about 10%, or less than about 5%, by number of lineage committed cells.

"Support" as used herein refers to any device or material that may serve as a foundation or matrix for the growth of adipose tissue-derived stromal stem cells.

"Therapeutic agent" or "therapeutic" refers to an agent capable of having a desired biological effect on a host. Chemotherapeutic and genotoxic agents are examples of therapeutic agents that are generally known to be chemical in origin, as opposed to biological, or cause a therapeutic effect by a particular mechanism of action, respectively. Examples of therapeutic agents of biological origin include growth factors, hormones, and cytokines. A variety of therapeutic agents are known in the art and may be identified by their effects. Certain therapeutic agents are capable of regulating cell proliferation and differentiation. Examples include chemotherapeutic nucleotides, drugs, hormones, non-specific (non-antibody) proteins, oligonucleotides (e.g., antisense oligonucleotides that bind to a target nucleic acid sequence (e.g., mRNA sequence)), peptides, and peptidomimetics.

A composition of the invention may include a substantially pure population of MSCs or the progeny thereof. The composition of the present invention may also include cell culture components, e.g., culture media including one or more of amino acids, metals and coenzyme factors. The composition may include small populations of other stromal cells. The composition may also include other non-cellular components which may support the growth and survival of the MSCs under particular circumstances, e.g. implantation, growth in continuous culture, or use as a biomaterial or composition.

The composition of the invention may comprise a population of cells in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are MSCs. In other words, in some embodiments at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells in the composition are MSCs.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, MSCs, either calculated by number, or by weight or by volume of the composition.

The MSCs may express one or more (*e.g.* two or more, three or more, four or more, five or more, six or more, seven or more, eight or more, nine or more, or ten or more) of the markers CD9, CD 10, CD13, CD29, CD44, CD49A, CD51, CD54, CD55, CD58, CD59, CD90 and CD105 at a significant level.

The MSCs may express one or more (*e.g.* two or more, three or more or four or more) of the markers CD9, CD44, CD54, CD90 and CD105, The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. The phenotypic surface marker characterization of a population of MSCs may be performed by any method known in the art. By way of example, but not limitation, this phenotypic characterization may be performed by individual cell staining. Such staining may be achieved through the use of antibodies. This may be direct staining, by using a labeled antibody or indirect staining, using a second labeled antibody against a primary antibody specific for the cell marker. Antibody binding may be detected by any method known in the art. Antibody binding may also be detected by flow cytometry, immunofluorescence microscopy or radiography.

Alternatively or additionally, a gene is considered to be expressed by a cell of the population of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in an adult stem cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

The MSC cell population used in the present invention may also be characterized in that the cells do not express a particular selection of markers at a detectable level. As defined herein, these markers are said be to be negative markers. In some embodiments, the stem cell population of the invention is considered not to express a marker if at least about 70% of the cells of the isolated adult stem cell population should not show detectable expression of the marker. In other embodiments, at least about 80%, at least about 90% or at least about 95% or at least about 97% or at least about 98% or at least about 99% or 100% of the cells of the stem cell population should not show any detectable expression of the marker. Again, lack of detectable expression may be proven through the use of an RT-PCR experiment or using FACS.

The composition of the invention may thus comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, six or more, seven or more, eight or more, nine or more, or ten or more) of the markers CD34, CD11b, CD14, CD15, CD16, CD31, CD34, CD45, CD49f, CD102, CD104, CD106 and CD133 at a significant level.

The composition of the invention may thus comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more *(e.g.* two or more, three or more, four or more, five or more, six or more, seven or more, or eight or more) of the markers CD11b, CD11c, CD14, CD31, CD34, CD45, CD133 and HLAII at a significant level.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which express one or more (*e.g.* two or more, or all three) of the markers c-Kit, vimentin and CD90 at a significant level. In one preferred embodiment, a composition of the invention comprises 95% or more cells with express c-Kit, vimentin and CD90 at a significant level.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, or all six) of the markers CD34, Factor VIII, alpha-actin, desmin, S-100 and keratin at a significant level.

The concentration of the MSCs in the composition of the invention may be at least about 1 x 10⁴ cells/mL, at least about 1 x 10⁵ cells/mL, at least about 1 x 10⁶ cells/mL, at least about 10 x 10⁶ cells/mL, or at least about 40 x 10⁶ cells/mL.

In some embodiments, at least about 40% (*e.g.* at least about 50%, at least about 60%, at least about 70%, at least about 80%, at least about 85%, at least about 90%, at least about 95% at least about 96%, at least about 97%, at least about 98%, or at least about 99%) of the MSCs in a composition of the invention are pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

In certain embodiments of the invention, the MSCs may be pre-stimulated using a concentration of IFN-γ between 0.1 and 100 ng/ml. In further embodiments, the MSCs may be pre-stimulated using a concentration of IFN-γ between 0.5 and 85 ng/ml, between 1 and 70 ng/ml, between 1.5 and 50 ng/ml, between 2.5 and 40 ng/ml, or between 3 and 30 ng/ml. Pre-stimulation may occur over a stimulation time longer than about 12 hours. Pre-stimulation may occur over a stimulation time longer than about 13 hours, longer than about 18 hours, longer than about 24 hours, longer than about 48 hours, or longer than about 72 hours.

In some embodiments of the invention, the MSCs may be characterized in that they:
a) do not express markers specific for antigen-presenting cells (APC),
b) do not express indoleamine 2,3-dioxygenase (IDO) constitutively, and
c) express IDO upon stimulation with interferon-gamma (IFN-γ).

MSCs useful in the context of the present invention have the ability to inhibit T cell activation. T cell activation can be measured by cytokine secretion and T cell proliferation. For example, MSCs of the invention preferably fail to elicit proliferation or secretion of IFNγ when co-cultured with unmatched PBMCs. MSCs of the invention may also inhibit secretion of IFNγ and the proliferation of PBMCs stimulation with the superantigen SEB.

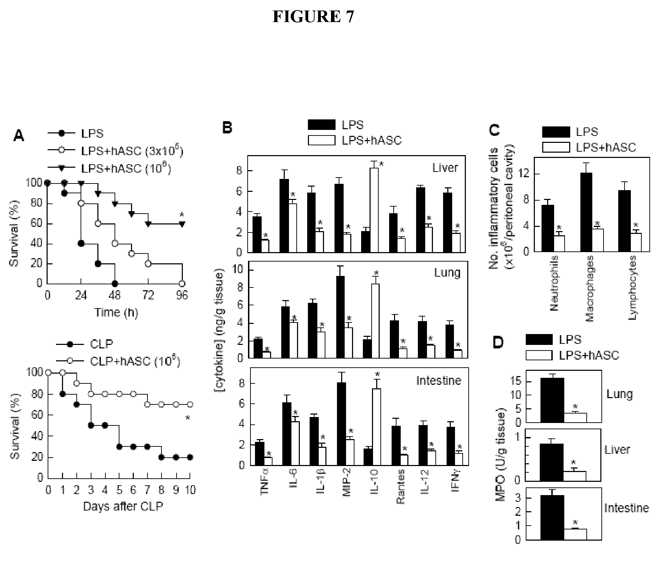

As is detailed in the examples, treatment with MSCs according to the invention (in this case, hASCs) were found to protect, in a dose-dependent manner, against mortality caused by endotoxin injection and cecal perforation (Figure 7A) in a mouse model of endotoxemia, and in this model also attenuated the clinical signs of septicemia, including lethargy, diarrhea, huddling, and piloerection (not shown). The pathogenesis of septic shock is characterized by overwhelmed inflammatory and immune responses that can lead to tissue damage, multiple organ failure, and death. Administration of hASCs to septic animals decreased the levels of inflammatory mediators and increased IL-10 in the major affected organs during septicemia (Figure 7B). Accordingly, MSCs used according to the present invention decrease inflammatory mediators, including one, two, three, four, five, six or all seven of TNFα, IL-6, IL-1β, IL-12, IFNγ, Rantes and MIP-2 in the major organs. MSCs used according to the invention preferably also significantly increase the level of IL-10 in the major organs during septicaemia. Treatment with MSCs should also significantly diminish the infiltration of inflammatory cells into the peritoneal cavity, lung, liver and intestine (see Figure 7C & 7D).

The results in the examples indicate that hASCs rescue mice from endotoxemic death by down-regulating the characteristic exacerbated inflammatory response of this disorder. The rescue of mice from endotoxemic death increases with increased number of cells administered. In this example at an administration of 1 million cells/mouse, 60% of the mice survived in contrast to none of the mice that received no cells. The survival percentage may increases when the number of cells injected into the mouse is increased. These examples provide evidence that MSCs, in particular hASCs, may be useful in the treatment of SIRS, sepsis, severe sepsis, septic shock and sepsis-like conditions.

A composition of the invention may contain the progeny of MSCs. Such progeny may include subsequent generations of MSCs, as well as lineage committed cells generated by inducing differentiation of the MSCs. Such differentiation may be induced *in vitro.* It will be understood that progeny cells may be obtained after any number of passages from the parental population. However, in certain embodiments, the progeny cells may be obtained after about 2, about 3, about 4, about 5, about 6, about 7, about 8, about 9, or about 10 passages from the parental population.

A composition of the invention may be provided under sterile conditions, and may be free of viruses, bacteria and other pathogens. A composition of the invention may be provided as a pyrogen-free preparation.

In one embodiment, a composition of the invention may be prepared for systemic administration (*e.g.* rectally, nasally, buccally, vaginally, via an implanted reservoir or via inhalation). In another embodiment, a composition of the invention may be prepared for local administration. A composition of the invention may be administered by the parenteral route. A composition may be administered by the subcutaneous, intracutaneous, intravenous, intramuscular, intra articular, intrasynovial, intrasternal, intrathecal, intralesional, intralymphatic and intracranial routes.

In one embodiment, the MSCs used in the invention may be autologous with respect to the subject to be treated. In a further embodiment, the MSCs used in the invention may be allogeneic or xenogeneic with respect to the subject to be treated. Previous studies have shown that allogeneic bone marrow-derived stromal stem cells and adipose tissue-derived stromal cells do not provoke a lymphocyte immune response when brought into contact with allogeneic lymphocytes *in vitro*. Consequently, allogenic adipose tissue-derived stromal stem cells derived from a donor may theoretically be used for the treatment of any patient, irrespective of MHC incompatibility. In embodiments wherein allogeneic stem cells are used, supportive treatment may be required. For example, immunosuppressants may be administered before, during and/or after treatment to prevent Graft-Versus-Host-Disease (GVHD), according to known methods. Prior to administration, the cells may also be modified to suppress an immune reaction from the subject to the cells or vice-versa, according to methods known in the art.

In one embodiment, the composition of the invention may be administered by injection or implantation of the composition at one or more target sites in the subject to be treated. In a further embodiment, the composition of the invention may be inserted into a delivery device which facilitates introduction of the composition into the subject by injection or implantation. In one embodiment the delivery device may comprise a catheter. In a further embodiment, the delivery device may comprise a syringe.

The compositions of the invention will generally comprise a pharmaceutically acceptable carrier and/or a diluent. Examples of such carriers and diluents are well known in the art, and may include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

A composition of the invention may be sterile and fluid to the extent that easy syringability exists. In addition, the composition may be stable under the conditions of manufacture and storage and preserved against the contaminating action of microorganisms such as bacteria and fungi through the use of, for example, parabens, chlorobutanol, phenol, ascorbic acid and thimerosal.

In one embodiment, the pharmaceutical composition of the invention may contain one or more (or two or more, or three or more, e.g. 1, 2, 3, 4 or 5) further therapeutic agents, such as a therapeutic agent selected from the following: an analgesic, such as a nonsteroidal anti-inflammatory drug, an opiate agonist or a salicylate; an anti-infective agent, such as an antihelmintic, an antianaerobic, an antibiotic, an aminoglycoside antibiotic, an antifungal antibiotic, a cephalosporin antibiotic, a macrolide antibiotic, a ß-lactam antibiotic, a penicillin antibiotic, a quinolone antibiotic, a sulfonamide antibiotic, a tetracycline antibiotic, an antimycobacterial, an antituberculosis antimycobacterial, an antiprotozoal, an antimalarial antiprotozoal, an antiviral agent, an anti-retroviral agent, a scabicide, an anti-inflammatory agent, a corticosteroid anti-inflammatory agent, an antipruritics/local anesthetic, a topical anti-infective, an antifungal topical anti-infective, an antiviral topical anti-infective; an electrolytic and renal agent, such as an acidifying agent, an alkalinizing agent, a diuretic, a carbonic anhydrase inhibitor diuretic, a loop diuretic, an osmotic diuretic, a potassium-sparing diuretic, a thiazide diuretic, an electrolyte replacement, and an uricosuric agent; an enzyme, such as a pancreatic enzyme and a thrombolytic enzyme; a gastrointestinal agent, such as an antidiarrheal, an antiemetic, a gastrointestinal anti-inflammatory agent, a salicylate gastrointestinal anti-inflammatory agent, an antacid anti-ulcer agent, a gastric acid-pump inhibitor anti-ulcer agent, a gastric mucosal anti-ulcer agent, a H2-blocker anti-ulcer agent, a cholelitholytic agent, a digestant, an emetic, a laxative and stool softener, and a prokinetic agent; a general anesthetic, such as an inhalation anesthetic, a halogenated inhalation anesthetic, an intravenous anesthetic, a barbiturate intravenous anesthetic, a benzodiazepine intravenous anesthetic, and an opiate agonist intravenous anesthetic; a hormone or hormone modifier, such as an abortifacient, an adrenal agent, a corticosteroid adrenal agent, an androgen, an anti-androgen, an immunobiologic agent, such as an immunoglobulin, an immunosuppressive, a toxoid, and a vaccine; a local anesthetic, such as an amide local anesthetic and an ester local anesthetic; a musculoskeletal agent, such as an anti-gout anti-inflammatory agent, a corticosteroid anti-inflammatory agent, a gold compound anti-inflammatory agent, an immunosuppressive anti-inflammatory agent, a non-steroidal anti-inflammatory drug (NSAID), a salicylate anti-inflammatory agent, a mineral; and a vitamins, such as vitamin A, vitamin B, vitamin C, vitamin D, vitamin E, and vitamin K.

In another embodiment, the further therapeutic agent may be a growth factor or other molecule that affects cell proliferation or activation. In a further embodiment that growth factor may induce final differentiation. In another embodiment, the growth factor may be a variant or fragment of a naturally-occurring growth factor. Methods of producing such variants are well known in the art, and may include, for example, making conservative amino acid changes, or by mutagenesis and assaying the resulting variant for the required functionality.

In one embodiment, MSCs may be administered to a subject in conjunction with one or more (or two or more, or three or more, e.g. 1, 2, 3, 4 or 5) further therapeutic agents. In some embodiments, the MSCs and the one or more further therapeutic agents may be administered to the subject simultaneously. In other embodiments, the MSCs and the one or more further therapeutic agents may be administered to the subject sequentially. The one or more further therapeutic agents may be administered before or after administration of the MSCs.

The dosage of MSCs and any further therapeutic agent will vary depending on the symptoms, age and body weight of the patient, the nature and severity of the disorder to be treated or prevented, the route of administration, and the form of the further therapeutic agent. The compositions of the invention may be administered in a single dose or in divided doses. Appropriate dosages for MSCs and any further therapeutic agent(s) may be determined by known techniques.

The precise time of administration and amount of any particular agent that will yield the most effective treatment in a given patient will depend upon the activity, pharmacokinetics, and bioavailability of the agent, the physiological condition of the patient (including age, sex, disease type and stage, general physical condition, responsiveness to a given dosage and type of medication), the route of administration, *etc*.. The information presented herein may be used to optimize the treatment, e.g., determining the optimum time and/or amount of administration, which will require no more than routine experimentation, such as monitoring the subject and adjusting the dosage and/or timing. While the subject is being treated, the health of the subject may be monitored by measuring one or more of relevant indices at predetermined times during a 24-hour period. Treatment regimens, including dosages, times of administration and formulations, may be optimized according to the results of such monitoring.

Treatment may be initiated with smaller dosages which are less than the optimum dose. Thereafter, the dosage may be increased by small increments until the optimum therapeutic effect is attained.

The combined use of several therapeutic agents may reduce the required dosage for any individual component because the onset and duration of effect of the different components may be complimentary. In such combined therapy, the different active agents may be delivered together or separately, and simultaneously or at different times within the day.

It will be apparent to one skilled in the art that the method for preparation of the composition of the invention is not limiting, and that compositions of the invention prepared in any way are included within the scope of the invention. In one embodiment, the invention provides a method of preparing a composition of the invention, which comprises: (a) collecting MSCs from a subject; (b) obtaining a cell suspension by enzymatic treatment; (c) sedimenting the cell suspension and re-suspending the cells in a culture medium; (d) culturing the cells for at least about 10 days; and (g) expanding the cells for at least two culture passages.

MSCs for use in the invention may be isolated from peripheral blood, bone marrow or adipose tissue of the subject into which the composition of the invention is to be introduced. However, the MSCs may also be isolated from any organism of the same or different species as the subject. Any organism with MSCs can be a potential candidate. In one embodiment the organism may be mammalian, and in another embodiment the organism is human.

In one preferred embodiment, adipose-derived MSCs can be obtained essentially as described by Zuk et al., 2001. According to this methodology, lipoaspirates are obtained from adipose tissue and the cells derived therefrom. In the course of this methodology, the cells may preferably be washed to remove contaminating debris and red blood cells, preferably with PBS. The cells are preferably digested with collagenase (e.g. at 37°C for 30 minutes, 0.075% collagenase; Type I, Invitrogen, Carlsbad, CA) in PBS. To eliminate remaining red blood cells, the digested sample can be washed (e.g. with 10% fetal bovine serum), treated with 160 mmol/L ClNH4, and finally suspended in DMEM complete medium (DMEM containing 10% FBS, 2 mmol/L glutamine and 1% penicillin/streptomycin). The cells can be filtered through a 40-µm nylon mesh. Cells isolated in this way can be seeded (preferably 2-3x104 cells/cm2) onto tissue culture flasks and expanded at 37°C and 5% CO2, changing the culture medium every 3-4 days. Cells are preferably passed to a new culture flask (1,000 cells/cm2) when cultures reach 90% of confluence.

In certain embodiments, the cells may be cultured for at least about 15 days, at least about 20 days, at least about 25 days, or at least about 30 days. The expansion of cells in culture may improve the homogeneity of the cell phenotype in the cell population.

In certain embodiments, the cells are expanded in culture for at least three culture passages or "passaged at least three times". In other embodiments, the cells are passaged at least four times, at least five times, at least six times, at least seven times, at least eight times, at least nine times, or at least ten times. The cells may be expanded in culture indefinitely provided that the homogeneity of the cell phenotype is improved and differential capacity is maintained.

Cells may be cultured by any technique known in the art for the culturing of stem cells. A discussion of various culture techniques, as well as their scale-up, may be found in Freshney, R.I., Culture of Animal Cells: A Manual of Basic Technique, 4th Edition, Wiley-Liss 2000. In certain embodiments, the cells are cultured by monolayer culture. Any medium capable of supporting MSCs in tissue culture may be used. Media formulations that will support the growth of MSCs include, but are not limited to, Dulbecco's Modified Eagle's Medium (DMEM), alpha modified Minimal Essential Medium (αMEM), and Roswell Park Memorial Institute Media 1640 (RPMI Media 1640). Typically, 0 to 20% Fetal Bovine Serum (FBS) will be added to the above media in order to support the growth of stromal cells. However, a defined medium could be used if the necessary growth factors, cytokines, and hormones in FBS for stromal cells and chondrocytes are identified and provided at appropriate concentrations in the growth medium. Media useful in the methods of the invention may contain one or more compounds of interest, including, but not limited to antibiotics, mitogenic or differentiating compounds for stromal cells. The cells of the invention may be grown at temperatures between 31°C to 37°C in a humidified incubator. The carbon dioxide content may be maintained between 2% to 10% and the oxygen content may be maintained at between 1% and 22%. Cells may remain in this environment for periods of up to about 4 weeks.

Antibiotics which can be added to the medium include, but are not limited to penicillin and streptomycin. The concentration of penicillin in the chemically defined culture medium may be about 10 to about 200 units per ml. The concentration of streptomycin in the chemically defined culture medium may be about 10 to about 200 ug/ml.

In one embodiment, the MSCs of the invention may be stably or transiently transfected or transduced with a nucleic acid of interest using a plasmid, viral or alternative vector strategy. Nucleic acids of interest include, but are not limited to, those encoding gene products which enhance the production of extracellular matrix components found in the tissue type to be repaired, e.g. intestinal wall or vaginal wall.

The transduction of viral vectors carrying regulatory genes into the stromal stem cells can be performed with viral vectors, including but not limited to adenovirus, retrovirus or adeno-associated virus purified (e.g. by cesium chloride banding) at a multiplicity of infection (viral units:cell) of between about 10:1 to 2000:1. Cells may be exposed to the virus in serum free or serum-containing medium in the absence or presence of a cationic detergent such as polyethyleneimine or Lipofectamine™ for a period of about 1 hour to about 24 hours (Byk T. et al. (1998) Human Gene Therapy 9:2493-2502; Sommer B. et al. (1999) Calcif. Tissue Int. 64:45-49).

Other suitable methods for transferring vectors or plasmids into stem cells include lipid/DNA complexes, such as those described in U.S. Pat. Nos. 5,578,475; 5,627,175; 5,705,308; 5,744,335; 5,976,567; 6,020,202; and 6,051,429. Suitable reagents include lipofectamine, a 3:1 (w/w) liposome formulation of the poly-cationic lipid 2,3-dioleyloxy-N-[2(sperminecarbox- amido)ethyl]-N,N-dimethyl-1-propanaminium trifluoroacetate (DOSPA) (Chemical Abstracts Registry name: N-[2-(2,5-bis[(3-aminopropyl)amino]-1-- oxpentyl}amino) ethyl]-N,N-dimethyl-2,3-bis(9-octadecenyloxy)-1-propanamin- ium trifluoroacetate), and the neutral lipid dioleoyl phosphatidylethanolamine (DOPE) in membrane filtered water. Exemplary is the formulation Lipofectamine 2000TM (available from Gibco/Life Technologies # 11668019). Other reagents include: FuGENETM 6 Transfection Reagent (a blend of lipids in non-liposomal form and other compounds in 80% ethanol, obtainable from Roche Diagnostics Corp. # 1814443); and LipoTAXITM transfection reagent (a lipid formulation from Invitrogen Corp., #204110). Transfection of stem cells can be performed by electroporation, e.g., as described in M.L. Roach and J.D. McNeish (2002) Methods in Mol. Biol. 185:1. Suitable viral vector systems for producing stem cells with stable genetic alterations may be based on adenoviruses and retroviruses, and may be prepared using commercially available virus components.

The transfection of plasmid vectors carrying regulatory genes into the MSCs can be achieved in monolayer cultures by the use of calcium phosphate DNA precipitation or cationic detergent methods (Lipofectamine™, DOTAP) or in three dimensional cultures by the incorporation of the plasmid DNA vectors directly into the biocompatible polymer (Bonadio J. et al. (1999) Nat. Med. 5:753-759).

For the tracking and detection of functional proteins encoded by these genes, the viral or plasmid DNA vectors may contain a readily detectable marker gene, such as the green fluorescent protein or beta-galactosidase enzyme, both of which can be tracked by histochemical means.

The invention will now be further illustrated by the following examples. These examples are provided by way of illustration only, and are not intended to be limiting.

### EXAMPLES

### 1. Materials and Methods - Cell Preparation

### 1.1 Mesenchymal stem cells (MSCs)

Human adipose-derived MSCs (hASCs) were obtained essentially as described (Zuk et al 2001), and prepared essentially as described in WO2006/037649.. Briefly, lipoaspirates obtained from human adipose tissue were washed twice with PBS to remove contaminating debris and red blood cells, and digested at 37°C for 30 minutes with 0.075% collagenase (Type I, Invitrogen, Carlsbad, CA) in PBS. The digested sample was washed with 10% fetal bovine serum (FBS), treated with 160 mmol/L ClNH₄ to eliminate remaining red blood cells, suspended in DMEM complete medium (DMEM containing 10% FBS, 2 mmol/L glutamine and 1% penicillin/streptomycin) and filtered through a 40-µm nylon mesh. Cells were seeded (2-3x10⁴ cells/cm²) onto tissue culture flasks and expanded at 37°C and 5% CO₂, changing the culture medium every 3-4 days. Cells were passed to a new culture flask (1,000 cells/cm²) when cultures reached 90% of confluence. A total of six different samples with population doublings 6-9 were used in the study.

### 1.1.1 Induction of indoleamine 2,3-dioxygenase (IDO) by interferon-gamma (IFN-γ)

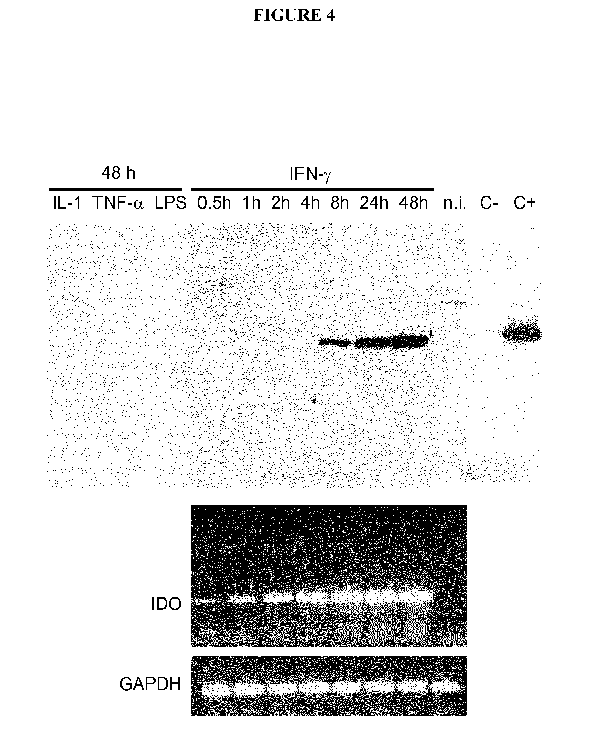

MSCs were isolated from human adipose tissue (ASCs), seeded onto tissue culture plates at a density of 10,000 cells/cm², and incubated for 48 hours in the conditions previously described for cell expansion. The following inflammatory stimuli were added to a portion of the cells, as shown in Figure 4:
- Interleukin-1 (IL-1): 3 ng/ml
- Interferon-gamma (IFN-γ) □: 3 ng/ml
- Tumor necrosis factor-alpha (TNF-α): 5 ng/ml
- Lipopolysaccharide (LPS): 100 ng/ml

The cells were incubated in the presence of the corresponding stimulus for a period ranging from 30 minutes to 48 hours, and were then collected by trypsin digestion, and lysed in RIPA buffer (50 mM Tris-HCl pH 7.4, 150 mM NaCl, 1 mM PMSF (phenyl-methylsulphonylfluoride), 1 mM EDTA (ethylenediaminetetraacetic acid), 5 µg/ml Aprotinin, 5 µg/ml Leupeptin, 1% Triton x-100, 1% Sodium deoxycholate, 0.1% SDS) containing protease inhibitors. Cell lysates were subjected to western blot analysis using an IDO-specific monoclonal antibody (mouse monoclonal IgG, clone 10.1, from *Upstate cell signaling solutions*). RNA was isolated from the treated cells, and analysed by reverse transcription - polymerase chain reaction (RT-PCR) using primers specific for the IDO cDNA (GenBank Accession No. M34455 (GI:185790))
forward 5' GGATTCTTCCTGGTCTCTCTATTGG 3'
reverse: 5' CGGACTGAGGGATTTGACTCTAATG 3'

### 1.1.2 Preparation of Stem Cells from Lipoaspirates with Improved Homogeneity

Cells were plated at low density in DMEM plus 10% FBS on glass cover slips in 24-well plates. Cells were washed with PBS and fixed in acetone for 10 min at -20° C. For staining of α-actin, cells were fixed in 4% paraformaldehyde for 10 min at RT. After blocking with PBS containing 4% goat serum and 0.1% Triton X-100, cells were incubated at 4°C overnight with primary antibodies against the following cell markers at the indicated dilutions: (i) alpha-actin; Dako, Glostrup, Denmark; 1/50; (ii) vimentin; Sigma, St. Louis, USA; 1/200; (iii) CD 90; CYMBUS, Biotechnology LTD, Chandlers Ford, Hants, UK; 1/50; (iv) Factor VIII; Dako; 1/100; (v) CD 34; Chemicon, CA, USA; 1/100; (vi) c-Kit; Chemicon; 1/100; (vii) desmin; Dako; 1/100; (viii) cytokeratin; Dako; 1/100 and (ix) S-100; Dako; 1/50. Then cells were incubated with the appropriate Fluorescein isothiocyanate (FITC)-conjugated or Tetramethylrhodamine isothiocyanate chloride (TRITC)-conjugated second antibodies (Sigma; 1/50) for 45 min at RT. Nuclei were counterstained with 4',6-diamidino-2-phenylindole (DAPI), cells were mounted in Mobiglow (MoBiTec, Gottingen, Germany) and observed with an epifluorescence microscope Eclipse TE300 (Nikon, Tokyo, Japan). In each case, the number of immunopositive cells was determined, and compared with the number of stained nuclei. Randomly selected fields were exported to a computer (MacIntosh G3; Apple Computer Inc., Cupertino, Ca, USA) through a Spotl camera (Diagnostic Instruments Inc., Tampa, FL, USA). Human aortic smooth muscle cells, human umbilical vein endothelial cells (HUVEC) and human synovial fibroblasts were used as positive controls for immunostaining with the various antibodies.

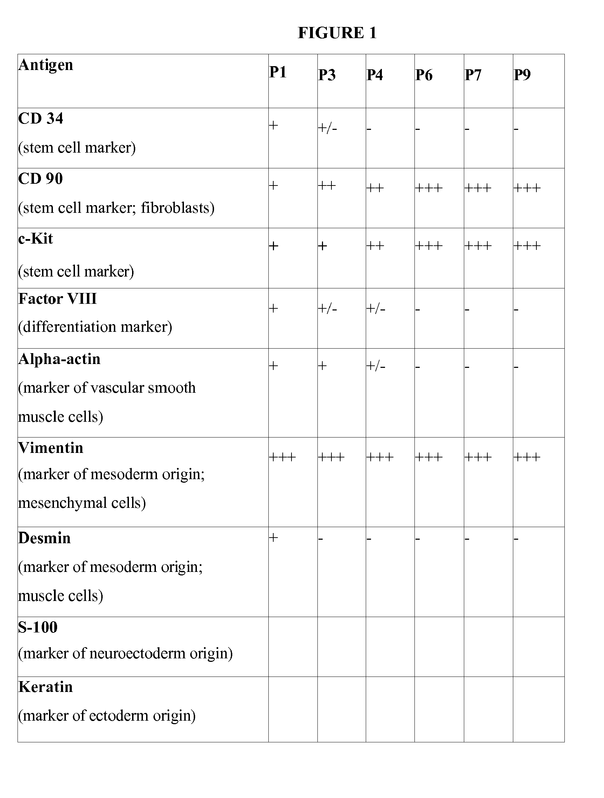

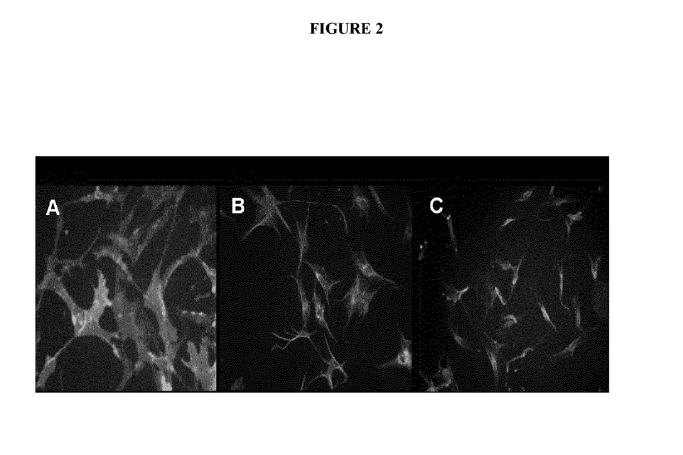

At passage 1, a high percentage (90-95%) of adipose-derived stromal stem cells expressed vimentin, a marker of mesenchymal cytoskeletal cells (Figure 1). Expression of vimentin was maintained at the same level up to and including passage 9. Levels of other markers fell over time. For example, α-actin, which was found in 17% of LPA-derived cells at passage 1 was no longer detectable at passage 7. The marker of endothelial cells, von Willebrandt factor (Factor VIII), and CD34, which is also found on the surface of endothelial cells, were only detected at passages 1 through 3 (7% and 12% immunopositive cells, respectively). By contrast, the expression of c-Kit (CD 117), a marker of cell proliferation, increased with time, with 99% immunopositive cells from passage 4 onwards (Figure 2). The fibroblast marker CD90, initially expressed in approximately 80% of LPA-derived cells, was found in 99% of cells from passage 6. No expression of the neuroectodermal marker S100 or the ectodermal marker keratin was observed in any of the LPA-derived cells at any time. The change in observed markers as the number of passages increases indicates an increase in the homogeneity of the cell preparation obtained.

To quantitate cell growth, cells were plated in 24-well plates at a concentration of 5 x10³ cells/cm².

After cells had attached to the substratum (3 h), the culture medium was replaced by DMEM supplemented with 1% antibiotics plus 0.5%, 2%, 5% or 10% FBS. As positive controls for testing of each batch of serum, human adipose-derived stromal stem cells were also cultured and their growth rates determined. Medium was replaced every two days. At 24h intervals, cells were fixed with 1 % glutaraldehyde and the number of cells per well was determined by nuclear staining with crystal violet, and monitoring absorbance at 595 nm. A standard curve was constructed to establish the relationship between cell number per well and absorbance at 595 nm (r²=0.99).

In order to analyze the cells in a more standardized and less subjective manner, the cells were also subjected to Fluorescence Activated Cell Sorter (FACS) analysis. In general, the flow cytometry analysis permits the detection of surface antigens by antibodies, which are directly (covalently) or indirectly (secondary fluorescent-labelled antibody) linked to a fluorescent marker. On the other hand, the above described immunohistochemical analysis requires permeabilization of the cells and subsequent antibody binding. Thus, the latter requires an individually optimized protocol depending on target protein and antibody. Moreover, due to the permeabilization of the cell membrane, it is not possible to distinguish between internally and externally expressed marker proteins.

The protocol used in the immunocytometry for the detection of surface antigens is standardized, and only requires appropriate negative controls. Further, the FACS analysis allows an evaluation of the percentage of positive cells and the level of expression. These evaluations are only of subjective nature using immunohistochemistry, and may vary from experiment to experiment, which does not occur with the FACS analysis.

Such immunophenotypic characterization of the cells may be performed on freshly isolated cells and after periods of cultures, for example, after 7 days, 4 weeks and 3 months in culture. The analysis of surface markers at different times allows assessment of the homogeneity of the phenotype during culturing. Examples of this analysis and data demonstrating the phenotype obtained from samples obtained from 3 healthy donors from zero to three months of culturing are described at length in U.S. Patent Application No. 11/065,461, filed on February 25, 2005, which is incorporated herein by reference.

After isolation by the above described method, the adipose-derived stromal stem cells from one of the patients were characterized by the presence/absence of a series of surface markers. To do this, the expression of the following surface markers was monitored by flow cytometry:
Integrin: CD11b, CD18, CD29, CD49a, CD49b, CD49d, CD49e, CD49f, CD51, CD61, CD104.
Hematopoietic markers: CD3, CD9, CD10, CD13, CD16, CD14, CD19, CD28, CD34, CD38, CD45, CD90, CD133, glycoforine.
Growth factor receptors: CD105, NGFR.
Extracellular matrix receptors: CD15, CD31, CD44, CD50, CD54, CD62E, CD62L, CD62P, CD102, CD106, CD146, CD166.
Others: CD36, CD55, CD56, CD58, CD59, CD95, HLA-1, HLA-II, β2-microglobulin.

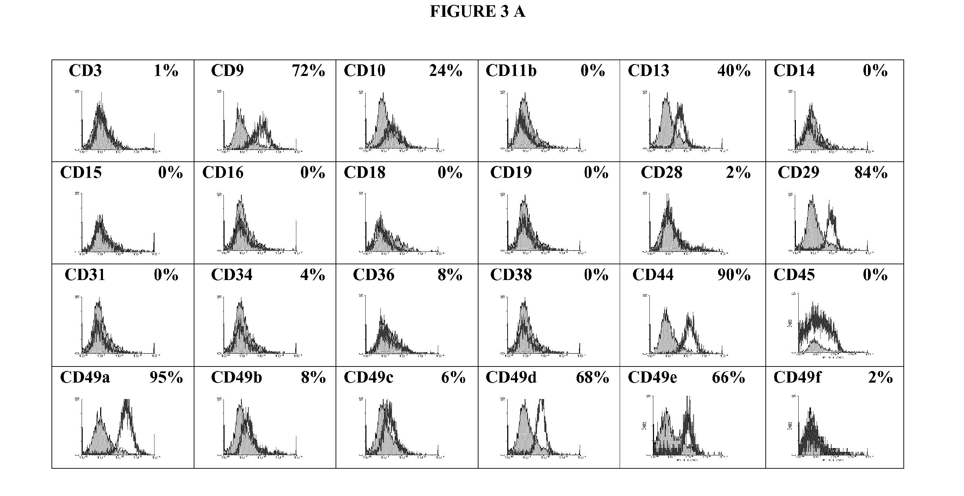

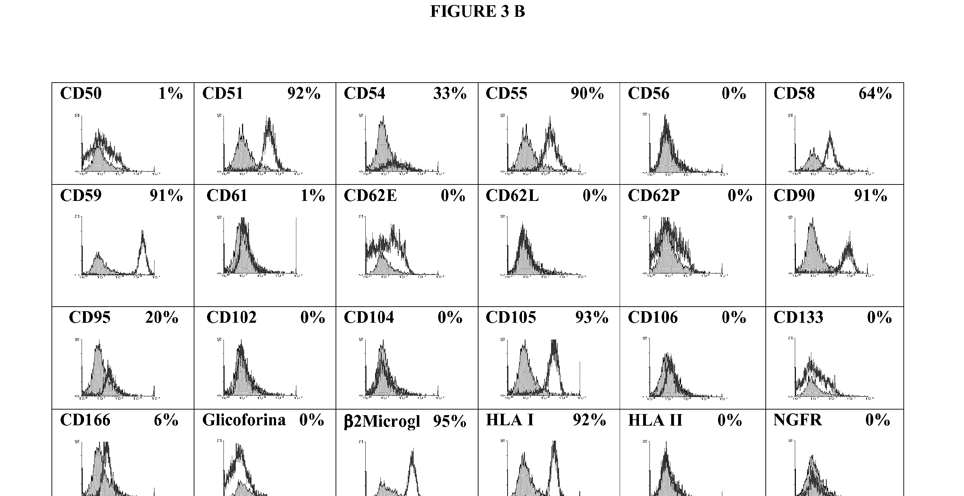

The cells to be characterized were collected by gentle digestion with trypsin, washed with PBS and incubated for 30 minutes at 4°C with fluorescein (FITC) or phycoerythrin (PE) labeled antibody markers against each of the surface markers to be analyzed. The cell markers were washed and immediately analyzed using the Epics-XL cytometer (Coulter). As controls, cells stained with unspecific antibodies of the corresponding isotopes labeled with FITC or PE. From the analysis of the expression profile of surface markers (Figure 3A/3B), the criteria used to determine which markers define the cell population and allow it to be identified and differentiated with respect to other types of cell were the following:
1. Discard those markers that vary from one sample to the other or over time during culturing in the experimentation done with healthy donors' adipose-derived stromal stem cells in the U.S. Patent Application No. 11/065,461, filed on February 25, 2005, which is incorporated herein by reference.
2. Select the markers as a function of their biological relevance, discarding markers characteristic of specific cell types (for example, CD3 is a marker exclusive to lymphocytes).

Applying these criteria, the multipotent stem cell population is characterized as being positive for expression of CD9, CD10, CD13, CD29, CD44, CD49A, CD51, CD54, CD55, CD58, CD59, CD90 and CD105; and lacking expression of CD11b, CD14, CD15, CD16, CD31, CD34, CD45, CD49f, CD102, CD104, CD106 and CD133.

### 1.2 Peripheral Blood Mononuclear Cells (PBMCs)

PBMCs were isolated from buffy coat preparations derived from the whole blood of healthy volunteers by density sedimentation on Ficoll-Hypaque gradients (20 min, 2000 rpm, at room temperature). Cells recovered from the gradient interface were washed twice in RPMI 1640 complete medium (consisting in RPMI 1640 medium supplemented with 8% human AB serum, 2 mM L-glutamine, 1% sodium pyuvate, 1% nonessential amino acids, 1% penicillin/streptomycin and 1% 2-mercaptoethanol) and immediately used for culture or further purification. To isolate T cells, PBMC were depleted of adherent cells by incubation with anti-CD8, -CD14, -CD19, -CD20 and -CD56 mAbs (all from Coulter Immunotech) for 1 h at 4°C, followed by incubation for 1 h at 4°C with anti-mouse IgG-coated magnetic beads. Bead-bound cells were removed from PBMC with a magnetic device. To minimize stimulation of cells, all the purification steps were carried out in the absence of serum. Purity of T cells was >96% as assessed by flow cytometry. CD4+ T cells were isolated by negative selection from the total PBMC using the CD4 isolation kit (Miltenyi Biotec), yielding a population of CD4+ cells with purity of 94-98%.

### 1.3 Cell culture

CD4+ T cells (5x104) and various numbers of either monocytes, DCs, or whole PBMCs were cultured in the presence or absence of the superantigen staphylococcal enterotoxin E (SEB, 1 ng/ml, Sigma), and in the presence or absence of indicated numbers of hASCs in flat-bottom 96-well plates (Coming, Coming, NY). After 72-96 h culture, cell proliferation was evaluated by using a cell proliferation assay (BrdU) from Roche Diagnostics GmbH (Mannheim, Germany). Cytokine content in culture supernatants was determined by specific sandwich ELISAs as below. Some cultures were performed in the presence of neutralizing antibodies against TGFβ (10 µg/mL) or IL-10 (10 µg/mL), indomethacin (20 µmoVL) or recombinant TNFα (20 ng/mL) or IFNγ (200 ng/mL) (all from BD Pharmingen, San Jose, CA and R&D Systems, Minneapolis, MN). To determine the cell-contact dependence of the suppressive response, SEB-stimulated PBMC (105) were placed in the upper insert of a Transwell system (Millipore, 0.4 µm pore), and hASCs (2x10⁴) in the absence or presence of irradiated (30 Gy) third-party PBMCs (5x10⁴) in the lower well. At day 4, the proliferative response of the PBMC in the upper compartment was determined. To produce supernatants from hASC cultures, hASCs (10⁵) were stimulated in a 25-cm² flask for 24 h with TNFα (20 ng/mL), IFNγ (200 ng/mL) or both, or for 4 days with allogeneic PBMCs (2x10⁶). The supernatants were collected, filtered through a 0.22 µm filter and added to SEB-activated PBMC cultures.

To measure the suppressive capacity of T cells generated in the presence of hASC, T cells were isolated from SEB-activated ASCs-PBMCs cultures after 4 days by positive immunomagnetic selection with magnetic bead-labeled anti-CD3 monoclonal antibodies (Miltenyi Biotec). Viable cells were recovered by density gradient centrifugation with Lymphoprep (Nycomed Pharma AS), rested for 2 days in RPMI complete medium supplemented with IL-2 (20 U/mL), and added at different ratios in a secondary culture to T cells stimulated with anti-CD3 Abs (5 µg/mL).

### 1.4 Characterization of the MSCs

Cell characterization was performed using cells at culture passages 1 to 25. Cells from adipose tissue were analyzed by means of flow cytometry using antibodies labeled with a fluorescent marker (i.e., by fluorescence immunocytometry) for the presence/absence of a series of surface markers, which included:
- Markers of antigen presenting cells (APCs): CD11b, CD11c, CD14, CD45, and HLAII.
- Markers of endothelial cells: CD31.
- Other markers: CD9, CD34, CD90, CD44, CD54, CD105 and CD133.

The antibodies used in the flow cytometry assay were the following:
- CD9: clone MM2/57 Mouse IgG2b - FITC labeled antibody (Serotec);
- CD11b: clone ICRF44 Mouse IgG1 - FITC labeled antibody (Serotec);
- CD11c: clone BU15 Mouse IgG1 - FITC labeled antibody (Serotec);
- CD14: clone UCHM1 Mouse IgG2a - FITC labeled antibody (Serotec);
- CD31: clone WM59 Mouse IgG1 - FITC labeled antibody (Serotec);
- CD34: clone QBEND 10 Mouse IgG1 - FITC labeled antibody (Serotec);
- CD44: clone F10-44-2 Mouse IgG2a - FITC labeled antibody (Serotec);
- CD45: clone F10-89-4 Mouse IgG2a - FITC labeled antibody (Serotec);
- CD54: clone 15.2 Mouse IgG1 - FITC labeled antibody (Serotec);
- CD90: clone F15-42-1 Mouse IgG1 - FITC labeled antibody (Serotec);
- CD105: clone SN6 Mouse IgG1 - FITC labeled antibody (Serotec); and
- Anti Human HLA class II DP, DQ, DR: clone WR18 Mouse IgG2a - FITC labeled antibody (Serotec);
- CD133: clone 293C3 Mouse IgG2b- PE labeled antibody (Miltenyi Biotec).

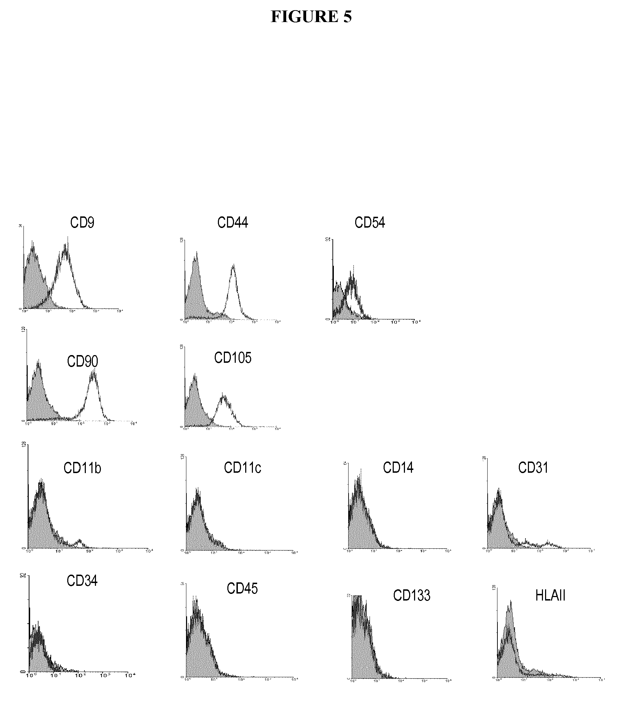

The cells analyzed (Figure 5) were positive for CD9, CD44, CD54, CD90 and CD105, and negative for CD11b, CD11c, CD14, CD31, CD34, CD45, CD133 and HLAII. The cells were negative for all of the tested markers which are specific for the endothelial or APC lineages (CD11b, CD11c, CD14, CD45, and HLAII).

### 1.5 Cytokine and hormone determination

For cytokine determination in colon mucosa, protein extracts were isolated by homogenization of colonic segments (50 mg tissue/mL) in 50 mmol/L Tris-HCl, pH 7.4, with 0.5 mmol/L DTT, and 10 µg/mL of a cocktail of proteinase inhibitors containing phenylmethylsulfonyl fluoride, pepstatin and leupeptin (Sigma). Samples were centrifuged at 30,000g for 20 min and stored at -80°C until cytokine determination. Cytokine, chemokine and HGF levels in the serum, colonic protein extracts and culture supernatants were determined by specific sandwich ELISAs using capture/biotinylated detection Abs from BD Pharmingen (San Diego, CA) and R&D Systems (Minneapolis, MN) according to the manufacture's recommendations. PGE₂ and HGF levels in culture supernatants were determined by using a competitive enzyme immunoassay kit (Cayman Chemical, Ann Arbor, MI). For intracellular analysis of cytokines in stimulated MLN cells, 10⁶ cells/ml were stimulated with PMA (10 ng/mL) plus ionomycin (20 ng/mL) for 8 h, in the presence of monensin. Cells were stained with PerCP-anti-CD4 mAbs for 30 min at 4°C, washed, fixed/saponin permeabilized with Cytofix/Cytoperm (Becton Dickinson), stained with 0.5 µg/sample of FITC- and PE-conjugated anti-cytokine specific mAbs (BD Pharmingen), and analyzed on a FACScalibur flow cytometer. In order to distinguish between monocyte/macrophage and T cell sources, intracellular cytokine analysis was done exclusively in the PerCP-labeled CD4 T cell population.

### 1.6 Myeloperoxidase assay

Neutrophil infiltration in the colon was monitored by measuring Myeloperoxidase (MPO) activity as previously described (Buras et al. (2005) Nature Reviews: Drug Discovery 4(10), 854 - 865). Briefly, colonic segments were homogenized at 50 mg/mL in phosphate buffer (50 mmol/L, pH 6.0) with 0.5% hexadecyltrimethylammonium bromide. Samples were frozen and thawed 3 times, centrifuged at 30,000g for 20 minutes. The supernatants were diluted 1:30 with assay buffer consisting in 50 mmol/L phosphate buffer pH 6.0 with 0.167 mg/mL o-dianisidine (Sigma) and 0.0005% H₂O₂, and the colorimetric reaction was measured at 450 nm between 1 and 3 min in a spectrophotometer (Beckman Instruments, Irvine, CA). MPO activity per gram of wet tissue was calculated as: MPO activity (U/g wet tissue) = (A₄₅₀) (13.5)/tissue weight (g), where A₄₅₀ is the change in the absorbance of 450nm light from 1 to 3 min after the initiation of the reaction. The coefficient 13.5 was empirically determined such that 1U MPO activity represents the amount of enzyme that will reduce 1 µmol peroxide/min.

### 1.7 Statistical analysis

All results are expressed as mean ± SD of *n* experiments or mice per group. The Mann-Whitney *U-*test to compare nonparametric data for statistical significance was applied on all clinical results and cell-culture experiments. Changes in the body weight were compared by using the Wilcoxon matched-pair signed-rank test. Survival was analyzed by the Kaplan-Meier log-rank test. P<0.05 was considered significant.

### 2. Example 1 - hASCs show potent immunumodulatory activities

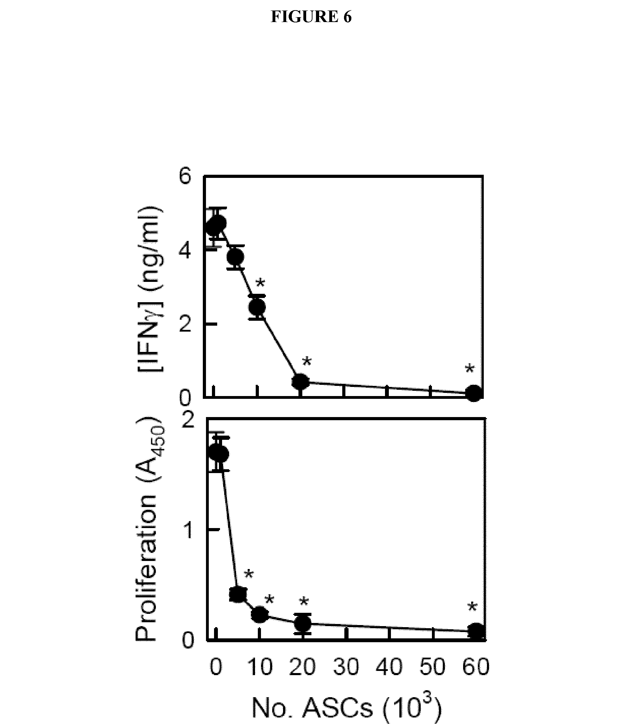

In this study the ability of the hASCs to inhibit T-cell activation was tested, as measured by cytokine secretion and T-cell proliferation. hASCs failed to elicit proliferation or secretion of IFNγ when co-cultured with unmatched PBMCs (not shown). Moreover, hASCs significantly inhibited the secretion of IFNγ and the proliferation of PBMCs stimulated with the superantigen SEB (Figure 6). This inhibitory effect directly correlated with the number of hASCs added to the co-culture (Figure 6), and was independent of the concentration of SEB (not shown).

### 3. Example 2 - Induction of SIRS by LPS and CLP (cecal ligation and puncture)

To induce endotoxemia, Balb/c mice were injected i.p. with LPS (400 µg/mouse). Mice were treated i.p. with medium or hASCs (10⁵-10⁶cells when indicated) 30 min after LPS injection.

Animals were monitored every 12 h for survival and other clinical signs including ruffled fur, lethargy, appearance of diarrhea, body weight loss. Some animals were sacrificed at different times after LPS injection, blood samples were collected by cardiac puncture, peritoneal exudates, liver, lungs and small intestines were collected. Tissue specimens were immediately frozen in liquid nitrogen for protein extraction and cytokine determination, and MPO activity measurement. The peritoneal suspension was centrifuged for 5 min at 1800g, and peritoneal cells were counted and adjusted in PBS/3 mmol/L EDTA medium to 3 x 10⁶ cells/mL. The number of viable cells in the different peritoneal subpopulations was determined by flow cytometry (FACScan; BD Biosciences, Mountain View, CA). Briefly, peritoneal lymphocytes, macrophages, and neutrophils were gated according to their different forward scatter and side scatter characteristics and counted.

In addition to the model of endotoxemia induced by high-dose LPS, the CLP model of peritonitis was used as this is considered to be the most reliable experimental model for human sepsis and a critical preclinical test for any new treatment of severe sepsis.

Treatment with hASCs protected in a dose-dependent manner against mortality caused by endotoxin injection and cecal perforation (Figure 7A) and attenuated the clinical signs of septicemia, including lethargy, diarrhea, huddling, and piloerection (not shown). The pathogenesis of septic shock is characterized by overwhelmed inflammatory and immune responses that can lead to tissue damage, multiple organ failure, and death. Administration of hASCs to septic animals decreased the levels of inflammatory mediators (TNFα, IL-6, IL-1β, IL-12, IFNγ, Rantes and MIP-2) and increased IL-10 in the major affected organs during septicemia (Figure 7B). Finally, treatment with hASCs diminished the infiltration of inflammatory cells into the peritoneal cavity, lung, liver and intestine (Figure 7C & 7D). These results indicate that hASCs rescue mice from endotoxemic death by down-regulating the characteristic exacerbated inflammatory response of this disorder.

These examples provide evidence that MSCs, in particular hASCs, may be useful in the treatment of SIRS, sepsis, severe sepsis, septic shock and sepsis-like conditions.

The majority of therapeutic strategies for SIRS, sepsis and septic shock to date have targeted pro-inflammatory mediators, and have not proved to be greatly successful in clinical trials. Therapies designed to block one single cytokine, e.g. TNFα or IL-1β, have shown limited efficacy due to the early and transient kinetic profile of these inflammatory cytokines. It has now been found that administration of mesenchymal stem cells (MSCs), in particular human adipose tissue derived stromal stem cells (hASCs), protects against mortality in two *in vivo* models of severe endotoxemia and sepsis, providing evidence that MSCs may be useful in the treatment of SIRS, sepsis and septic shock. It has been found that MSCs function at several levels to regulate crucial aspects of SIRS, including by reduction of systemic levels of various inflammatory cytokines and chemokines, and by inhibition of leukocyte infiltration into various target organs.

Embodiments of the invention include:
1. A composition comprising mesenchymal stem cells (MSCs), for use in treating systemic inflammatory response syndrome (SIRS) in a subject.
2. Use of mesenchymal stem cells (MSCs) in the manufacture of a medicament for treating systemic inflammatory response syndrome (SIRS) in a subject.
3. A method of treating systemic inflammatory response syndrome (SIRS) in a subject, comprising administering mesenchymal stem cells (MSCs) to the subject.
4. The composition, use or method according to any of embodiments 1-3, wherein the SIRS is sepsis, severe sepsis, septic shock or a sepsis-like condition.
5. The composition, use or method according to embodiment 4, wherein the sepsis, severe sepsis, or septic shock is caused by a virus, fungus, or protozoan.
6. The composition, use or method according to embodiment 4, wherein the sepsis, severe sepsis, or septic shock is caused by a bacterium.
7. The composition, use or method according to any of the preceding embodiments, wherein the MSCs are adipose tissue derived stromal stem cells (ASCs).
8. The composition, use or method according to any of embodiments 1-6 wherein the MSCs are bone marrow stem cells (BM-MSCs).
9. The composition, use or method according to any of the preceding embodiments, wherein at least about 50% of the MSCs express one or more of the markers CD9, CD10, CD13, CD29, CD44, CD49A, CD51, CD54, CD55, CD58, CD59, CD90 and CD105.
10. The composition, use or method according to embodiment 9, wherein at least about 50% of the MSCs express the markers CD9, CD10, CD13, CD29, CD44, CD49A, CD51, CD54, CD55, CD58, CD59, CD90 and CD105.
11. The composition, use or method according to any of the preceding embodiments, wherein at least about 50% of the MSCs do not express one or more of the markers CD11b, CD14, CD15, CD16, CD31, CD34, CD45, CD49f, CD102, CD104, CD106 and CD133.
12. The composition, use or method according to embodiment 11, wherein at least about 50% of the MSCs do not express the markers CD11b CD14, CD15, CD16, CD31, CD34, CD45, CD49f, CD102, CD104, CD106 and CD133.
13. The composition, use or method according to any of the preceding embodiments wherein the MSCs are characterised in that they:
   a) do not express markers specific for antigen-presenting cells (APC);
   b) do not express indoleamine 2,3-dioxygenase (IDO) constitutively; and
   c) express IDO upon stimulation with interferon-gamma (IFN-γ).
14. The composition, use or method according to any of the preceding embodiments, wherein the MSCs are administered in a pharmaceutically acceptable carrier and/or a diluent.
15. The composition, use or method according to any of preceding embodiments, wherein the MSCs are administered systemically.
16. The composition, use or method according to embodiment 15 wherein the MSCs are administered rectally, nasally, buccally, vaginally, via an implanted reservoir or via inhalation.
17. The composition, use or method according to any one of embodiments 1-14 wherein the MSCs are administered locally.
18. The composition, use or method of embodiment 17 wherein the MSCs are administered by injection or implantation.
19. The composition, use or method according to any preceding embodiment wherein the MSCs are administered via the subcutaneous, intracutaneous, intravenous, intramuscular, intraarticular, intrasynovial, intrasternal, intrathecal, intralesional, or intracranial route.
20. The composition, use or method according to any one of the preceding embodiments wherein the MSCs are autologous with respect to the subject to be treated.
21. The composition, use or method according to any one of embodiments 1-19 wherein the MSCs are allogeneic with respect to the subject to be treated.
22. The composition, use or method according to embodiment 21 wherein an immunosuppressant is administered to the subject before, during or after treatment.
23. The composition, use or method according to embodiment 21 or 22 wherein the MSCs are pretreated to suppress an immune reaction.
24. The composition, use or method according to any of the preceding embodiments, wherein the MSCs are administered in conjunction with one or more further therapeutic agents.
25. The composition, use or method according to embodiment 24, wherein the MSCs and the one or more further therapeutic agents are administered to the subject simultaneously.
26. The composition, use or method according to embodiment 24, wherein the MSCs and the one or more further therapeutic agents are administered to the subject sequentially.
27. The composition, use or method according to embodiment 26, wherein the MSCs are administered to the subject before the one or more further therapeutic agents.
28. The composition, use or method according to embodiment 26, wherein the MSCs are administered to the subject after the one or more further therapeutic agents.
29. The composition, use or method according to any of embodiments 24-28 wherein the one or more further therapeutic agents are selected from the group consisting of an analgesic, an anti-infective agent, an electrolytic or renal agent, an enzyme, a gastrointestinal agent, a general anesthetic, a hormone or hormone modifier, an immunobiologic agent, a local anesthetic, a musculoskeletal agent, a growth factor or other molecule that affects cell proliferation or activation or induces final differentiation, and a fragment or variant thereof.
30. A catheter or syringe containing the composition of any of the preceding embodiments, for use in treating systemic inflammatory response syndrome (SIRS) in a subject.

## Claims

1. A composition comprising mesenchymal stem cells (MSCs), for use in treating systemic inflammatory response syndrome (SIRS) in a subject.

2. The composition for use according to claim 1, wherein the SIRS is sepsis, severe sepsis, septic shock or a sepsis-like condition.

3. The composition for use according to claim 2, wherein the sepsis, severe sepsis, or septic shock is caused by a virus, fungus, or protozoan.

4. The composition for use according to claim 2, wherein the sepsis, severe sepsis, or septic shock is caused by a bacterium.

5. The composition for use according to any one of the preceding claims, wherein the MSCs are adipose tissue derived stromal stem cells (ASCs).

6. The composition for use according to any one of claims 1-4, wherein the MSCs are bone marrow stem cells (BM-MSCs).

7. The composition for use according to any one of claims 1-6, wherein the MSCs are expanded in culture for at least three culture passages.

8. The composition for use according to any one of the preceding claims, wherein at least about 50% of the MSCs express one or more of the markers CD9, CD10, CD 13, CD29, CD44, CD49A, CD51, CD54, CD55, CD58, CD59, CD90 and CD105.

9. The composition for use according to any one of the preceding claims, wherein at least about 50% of the MSCs do not express one or more of the markers CD11b CD11c, CD14, CD15, CD16, CD31, CD34, CD45, CD49f, CD102, CD104, CD106, CD133 and HLAII.

10. The composition for use according to any one of the preceding claims, wherein the MSCs are administered systemically.

11. The composition for use according to any one of claims 1-9, wherein the MSCs are administered locally.

12. The composition for use according to any one of the preceding claims, wherein the MSCs are administered via the subcutaneous, intracutaneous, intravenous, intramuscular, intraarticular, intrasynovial, intrasternal, intrathecal, intralesional, or intracranial route.

13. The composition for use according to any one of claims 1-12, wherein the MSCs are allogeneic with respect to the subject to be treated.

14. The composition for use according to any one of the preceding claims, wherein the MSCs are administered in conjunction with one or more further therapeutic agents.

15. The composition for use according to claim 14, wherein the one or more further therapeutic agents are selected from the group consisting of an analgesic, an anti-infective agent, an electrolytic or renal agent, an enzyme, a gastrointestinal agent, a general anesthetic, a hormone or hormone modifier, an immunobiologic agent, a local anesthetic, a musculoskeletal agent, a growth factor or other molecule that affects cell proliferation or activation or induces final differentiation, and a fragment or variant thereof.
